# EUROPEAN PATENT APPLICATION

(11) **EP 0 974 354 A1**
(43) Date of publication of application: **26.01.2000**
(21) Application number: 98305594.8
(22) Date of filing: 14.07.1998
(51) Int. Cl.: A61K 31/35, C07D 493/22

(54) **Artemisinin derivatives**

(71) Applicant: The Hong Kong University of Science & Technology, Kowloon (HK)
(72) Inventor: Haynes, Richard Kingston, Kowloon (HK); Lam, Wai-Lun, Kowloon (HK); Chan, Ho-Wai, Shatin, New Territories, (HK); Tsang, Hing-Wo, Sha Tin, New Territories (HK)
(74) Representative: Wallace, Sheila Jane

(57) **Abstract**

This invention relates to the use of certain C-10 ether-substituted derivatives of artemisinin in the treatment and/or prophylaxis of diseases caused by infection with a parasite, certain novel C-10 ether-substituted derivatives of artemisinin, processes for their preparation and pharmaceutical compositions containing such C-10 ether-substituted derivatives. The compounds are particularly effective in the treatment of malaria, neosporosis and coccidiosis.

## Description

This invention relates to the use of certain C-10 ether-substituted derivatives of artemisinin in the treatment and/or prophylaxis of diseases caused by infection with a parasite, certain novel C-10 ether-substituted derivatives of artemisinin, processes for their preparation and pharmaceutical compositions containing such C-10 ether-substituted derivatives.

Malaria is the most important human parasitic disease in the world today. Approximately 270 million people throughout the world are infected with malaria, with about 2 million dying each year. The ability of parasites to produce a complex survival mechanism by expressing variant antigens on the surface of infected erythrocytes makes it possible for the parasites to escape from the destructive action of the host immune response against these antigens. In addition, the increasing rate of malaria infection is due to the spread of chloroquine-resistant strains of Plasmodium falciparum and the other multi-drug resistant strains.

In the field of animal health, parasitic diseases are a major problem, especially those diseases which are functionally related to malaria. For instance, neosporosis is a term used to describe diseases caused by parasites of the species Neospora, especially Neospora caninum, in animals. Neospora infections are known to occur in dogs, cattle, sheep, goats and horses.

The final host for Neospora spp., including Neospora caninum, is unknown and, in addition, the complete cycle of development of the parasite is not understood. The asexual phases of reproduction, known as schizogony, and the behaviour of the unicellular tachyzoite/bradyzoite stage have been clarified, however. Tachyzoites are infectious unicellular parasite stages of about 3-7 x 1-5 mm in size formed after intracellular reproduction termed endodyogeny. Reproduction via tachyzoites takes place preferentially in organelles such as muscle or nerve cells. Pathological symptoms invoked after an infection are associated mainly in those tissues. Some five to six weeks after natural infection in a dog, symptoms of the disease are hypersensitivity caused by inflammation of neuronal cells and increasing tendency to hyperextension of the hind legs. Histopathological lesions are apparent in the nervous system, preferentially in the brain and spinal cord. Extensive non-suppurative inflammations, glial excrescences and perivascular infiltrations of mononuclear cells (macrophages, lymphocytes, plasma cells) dominate, and are also partly apparent in eosinophils and neutrophils. In the muscular system, macroscopically observable necroses and degenerative changes appear. Apart from the more or less strongly developed atrophy, long pale longitudinal stripes are evident.

In California and Australia, Neospora caninum infections appear to be the main cause for abortion in cattle. Symptoms of the disease in cattle are similar to those in the dog. Ataxia is apparent, joint reflexes are weakened and pareses at the hind legs, partly in all four legs, can be observed. The histological picture is similar to that of the dog; mainly non-suppurative meningitis and myelitis.

Data on in vivo activity of compounds suitable against neosporosis are rare because adequate in vivo test systems still have to be developed. Sulfadiazin (administered via drinking water) is effective in experimentally infected mice, only if the treatment was prophylactic, that is, the treatment was started before infection. In dogs, treatment with sulfadiazin and clindamycin is only successful if it is started early, that is, at the appearance of first clinical symptoms as a result of neuronal inflammation.

Coccidiosis, an infection of the small intestine, is relatively rarely diagnosed in humans, where it is caused by Isospora belli. However, humans are also the final host of at least two cyst-forming coccidial species (Sarcocystis suihominis and S. bovihominis). Consumption of raw or inadequately cooked pork or beef containing such cysts can lead to severe diarrhoea, the cause of which is probably seldom diagnosed correctly. Coccidia (phylum Apicomplexa, suborder Eimeriina) are one of the most successful groups of parasitic protozoans, having conquered virtually every class of Metazoa. The ones that are of particular importance for man are the 60-100 species which parasitise domestic animals and which in some instances can cause very severe losses, especially in poultry, although also in lambs, calves, piglets, rabbits and other animals (see Table A).

**Table A:**

| Causatives of intestinal coccidiosis in domestic animals | | |
|---|---|---|
| Animal | number of Eimeria and/or Isospora species*) | most pathogenic and/or very common species (E=Eimeria, I=Isospora) |
| chicken (Gallus gallus) | 7 | E.tenella, E.necatrix, E.maxima, E.acervulina |
| turkey (Meleargidis gallopavo) | 7 | E.meleagrimitis, E.adenoides |
| goose (Anser anser) | 6 | E.anseris, E.truncata, E.nocens, E. kotlani |
| duck (Anas platyhynehus) | 3 | Tyzzeria perniciosa, E.anatis |
| pigeon (Columba livia) | 2 | E.columbarum, E.labbeanea |
| rabbit (Oryctolagus cuniculus) | 11(12) | E.intestinalis, E.flavescens, E.stiedai, E.magna, E.perforans |
| sheep (Ovis arius) | 11(16) | E.ovinoidalis,E.ashataE.o vina |
| goat (Capra hircus) | 12(15) | E.ninakohlyakimovae, E.arloingi |
| cattle (Bos taurus) | 12(15) | E.zuernii, E.bovis, E.auburnensis |
| pig (Sus scofra) | 7(14) | I.suis, E.debliecki, E.scabra |
| dog (Canis familiaris) | 5 | I.canis, I.(Cystisospora) burrowsi |
| cat (Felis catus) | 2+6 | I.felis, I.rivolta as final host: Sarcocystis bovifelis, S.ovifelis, S.fusiformis, S.muris, S.cuniculi, Toxoplasma gondii |

| | | |
|---|---|---|
| *) regarding to Pellerdy (1974), Eckert et al, (1995b, Levine and Ivens (1970) and Mehlhorn 1988) | | |

Most of the pathogenic species are strictly host-specific. They have a complex life cycle with two asexual reproduction phases (schizogony or merogony, and sporogony) and a sexual development phase (gametogony). In view of the major importance of coccidiosis, numerous reviews are available, for instance, by Davies et al. (1963), Hammond and Long (1973), Long (1982, 1990), and Pellerdy (1974). The economically important species sometimes differ very considerably in their sensitivity to medicinal active ingredients. The sensitivity of the different developmental stages to medicinal agents also varies enormously.

As far as the use of drugs is concerned, prophylaxis is the main approach in poultry, in which symptoms do not appear until the phase of increased morbidity, and therapy is the principal strategy in mammals (McDougald 1982). Polyether antibiotics and sulfonamides, among other drugs, are currently used for such treatment and prophylaxis. However, drug-resistant strains of Eimeria have emerged and drug-resistance is now a serious problem. New drugs are therefore urgently required. Given the multiplicity of pathogens and hosts, there is no "ideal model" for identifying and testing anticoccidial agents. For example, most of the many substances used for preventing coccidiosis in poultry are insufficiently effective or even completely ineffective against mammalian coccidia (Haberkorn and Mundt; 1989; Haberkorn 1996). Numerous works and sets of instructions have been published on testing of active ingredients in animals for anticoccidial efficacy, for immunisation, etc. One particularly important and comprehensive example is the survey of current methods published by Eckert et al. (1995a).

The compound artemisinin, also known as qinghaosu (1), is a tetracyclic 1,2,4-trioxane occurring in Artemisia annua. Artemisinin and its derivatives dihydroartemisinin (2), artemether (3) and sodium artesunate (4) have been used for the treatment of malaria.

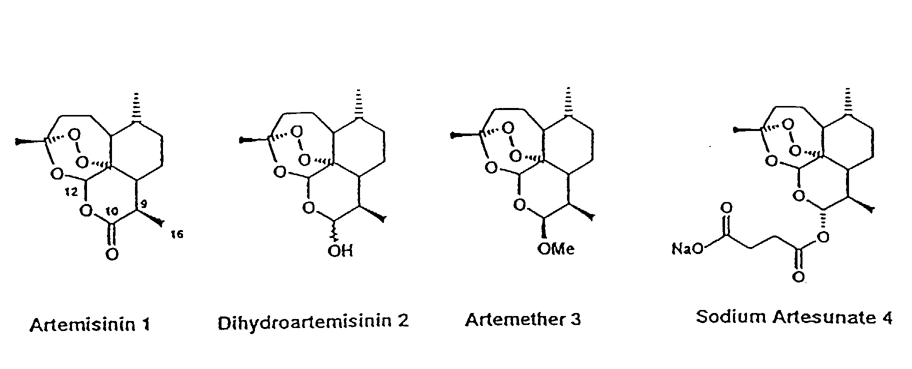

Different modes of action have been proposed by various groups to account for the action of artemisinin and its derivatives in treating malaria (Posner et al., *J.Am.Chem.Soc.**1996**,118,3537*;Posner at al., *J.Am.Chem.Soc.**1995**,117, 5885*;Posner at al., *J. Med.Chem.**1995**,38,2273).* However, irrespective of actual mode of action, all current derivatives suffer from poor oral bioavailability and poor stability (Meshnick et al., *Parasitology Today **1996**,12,79),* especially the 'first generation' ethers and esters artemether and sodium artesunate obtained from dihydroartemisinin. Extensive chemical studies carried out on artemisinin and derivatives indicate that a cause of instability is the facile opening of the trioxane moiety in artemisinin itself, or in the metabolite common to all currently used derivatives artemether, arteether and artesunate, namely dihydroartemisinin. Ring opening will provide the free hydroperoxide, which is susceptible to reduction. Removal of this group ensures destruction of drug activity with the reduction products being transformed into desoxo metabolites. In order to render ring-opening less facile, the oxygen atom at C-10 can be either removed to provide 10-deoxydihydroartemisinin, or replaced by other groups, and this has provided the basis for the so-called 'second generation' compounds which are generally 10-deoxy artemisinin derivatives. In addition, derivatives of artemisinin have also been prepared with a variety of substituents at C-9.

Artemisinin derivatives are also known in which the oxygen atom at C-10 forms part of a variety of ether groups. For instance, Li et al (Acta Pharmaceutical Sinica,1981,16,429) synthesised, inter alia, ether derivatives of artemisinin in which the C-10 carbon atom is substituted by a group of formula -O-R^{A} where R^{A} represents a methyl, ethyl, n-propyl, isopropyl, n-butyl, isopentyl, tert-pentyl, n-octyl, 2-hydroxyethyl, 2-methoxyethyl, prop-2-enyl, 3-phenylprop-2-enyl, cyclopentyl, cyclohexyl, benzyl or 4-methoxybenzyl group and evaluated these compounds for activity against a chloroquine-resistant strain of Plasmodium berghei. Ramu and Baker (J.Med.Chem.1995, 38,1911-1921) synthesised glucuronide tri-ethanoyloxy ester derivatives of dihydroartemisinin and hydrolysed such derivatives to form the α- and β-dihydroartemisininglucuronides. The latter compounds exhibited in vitro activity against Plasmodium falciparum. Vishwakarma et al (J.Nat.Prod.,1992, 55(8),1142-1144) described a stereoselective synthesis of α-artelinic acid and its methyl ester and sodium salt and tested the acid for antimalarial activity against Plasmodium knowlesi in vivo. This article also refers to β-artelinic acid and its potassium salt. Lin et al (J.Med.Chem.,1990,33,2610-2614) referred to their earlier synthesis of β-artelinic acid and its sodium salt and the antimalarial activity of these compounds in mice infected with Plasmodium berghei.

US Patent No. 5486535 describes the activity of dihydroartemisinin and ether derivatives of artemisinin in which the C-10 carbon atom is substituted by a group of formula -O-R^{B} where R^{B} represents a methyl, ethyl, iso-propyl, iso-butyl or see-butyl group against Toxoplasma gondii.

US Patent No. 5225427 discloses certain 10-substituted ether derivatives of artemisinin in which the C-10 carbon atom is substituted by a group of formula -O-R^{C} where R^{C} represents a 1-ethanoylethyl, 1,3-bis(isopropoxy)prop-2-yl, but-3-yn-2-yl, 2-methylbut-3-yn-2-yl, 2-ethylbut-3-yn-2-yl, 2-(4-chlorophthalimido)ethyl, 3-(4-carboxylphenyl)-isoxazol-5-ylmethyl, 3-chloroisoxazol-5-ylmethyl or 3-bromoisoxazol-5-yl methyl group. The activity of all these compounds against Plasmodium berghei was demonstrated in vivo and the 2-ethylbut-3-yn-2-yl and 3-(4-carboxylphenyl)isoxazol-5-ylmethyl derivatives were also shown to possess antiamoebic activity against Entamoeba histolytica in vitro.

Whilst the current artemisinin derivatives are successful, there are problems associated with stability, bioavailability and potential neurotoxicity. There is also a need for artemisinin derivatives which exhibit a broad spectrum of activity against a variety of parasites.

It has now been discovered that certain C-10 ether-substituted derivatives of artemisinin are effective in the treatment of diseases caused by infection with a parasite. These compounds are particularly effective in the treatment of malaria, neosporosis and coccidiosis, especially forms of these diseases caused by infection with a parasite of the genera Plasmodium, Neospora or Eimeria, particularly Plasmodium falciparum, Neospora caninum and Eimeria tenella. According to the present invention there is therefore provided a compound of the general formula I 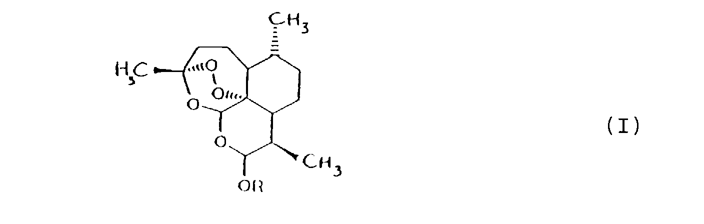 or a salt thereof
in which
R represents a hydrogen atom or an optionally substituted alkyl, alkenyl, alkynyl, cycloalkyl, aryl, aralkyl, heterocyclic or polycyclic group;
for use in the treatment and/or prophylaxis of a disease caused by infection with a parasite other than an organism of a genus selected from the genera Toxoplasma, Entamoeba and Plasmodium.

Suitable salts include metal salts of compounds in which the substituent R bears a terminal carboxyl group. Such metal salts are preferably formed with an alkali metal atom such as a lithium, sodium or potassium atom. Sodium salts are particularly preferred.

Any alkyl, alkenyl or alkynyl group, unless otherwise specified, may be linear or branched and may contain up to 12, preferably up to 6, and especially up to 4 carbon atoms. Preferred alkyl groups are methyl, ethyl, propyl and butyl. It is preferred that any alkenyl or alkynyl group is not an alk-1-enyl or alk-1-ynyl group. In other words, there should preferably be at least one methylene group -CH₂- or similar sp³-hybridised centre between a carbon atom forming part of the double or triple C-C bond and the oxygen atom to which the group is attached. Preferred alkenyl and alkynyl groups are propenyl, butenyl, propynyl and butynyl groups. When an alkyl moiety forms part of another group, for example the alkyl moiety of an aralkyl group, it is preferred that it contains up to 6, especially up to 4, carbon atoms. Preferred alkyl moieties are methyl and ethyl.

An aryl group may be any aromatic hydrocarbon group and may contain from 6 to 24, preferably 6 to 18, more preferably 6 to 16, and especially 6 to 14, carbon atoms. Preferred aryl groups include phenyl, naphthyl, anthryl, phenanthryl and pyryl groups, especially phenyl and naphthyl groups. When an aryl moiety forms part of another group, for example the aryl moiety of an aralkyl group, it is preferred that it is a phenyl, naphthyl, anthryl, phenanthryl or pyryl, especially a phenyl or naphthyl, moiety.

An aralkyl group may be any alkyl group substituted by an aryl group. A preferred aralkyl group contains from 7 to 30, particularly 7 to 24 and especially 7 to 18, carbon atoms, particularly preferred aralkyl groups being benzyl, naphthylmethyl, anthrylmethyl, phenanthrylmethyl and pyrylmethyl groups, a benzyl group being especially preferred.

A cycloalkyl group may be any saturated cyclic hydrocarbon group and may contain from 3 to 12, preferably 3 to 8, and especially 3 to 6, carbon atoms. Preferred cycloalkyl groups are cyclopropyl, cyclopentyl and cyclohexyl groups.

A polycyclic group may be any saturated or partially unsaturated hydrocarbon group which contains more than one ring system. Such ring systems may be "fused", that is, adjacent rings have two adjacent carbon atoms in common, "bridged", that is, the rings are defined by at least two common carbon atoms (bridgeheads) and at least three acyclic chains (bridges) connecting the common carbon atoms, or "spiro" compounds, that is, adjacent rings are linked by a single common carbon atom. It is also envisaged that a polycyclic group may contain more than one of these types of ring system. Polycyclic groups preferably contain from 4 to 30, particularly 4 to 26, and especially 6 to 18, carbon atoms. Bicyclic, tricyclic and tetracyclic groups are particularly preferred. Preferred bicyclic groups contain from 4 to 14, especially 6 to 10, carbon atoms, preferred tricyclic groups contain from 5 to 20, especially 6 to 14, carbon atoms, and preferred tetracyclic groups contain from 6 to 26, especially 6 to 18, carbon atoms. Cholestanyl and cholestenyl groups are particularly preferred polycyclic groups.

A heterocyclic group may be any monocyclic or polycyclic ring system which contains at least one heteroatom and may be unsaturated or partially or fully saturated. The term "heterocyclic" thus includes aromatic heterocyclic groups termed "heteroaryl" groups as well as non-aromatic heterocyclic groups. A heteroaryl group may be any aromatic monocyclic or polycyclic ring system which contains at least one heteroatom. Preferably, a heteroaryl group is a 5- to 18- membered, especially a 5- to 14-membered, aromatic ring system containing at least one heteroatom selected from oxygen, sulphur and nitrogen atoms. Preferred heteroaryl groups include pyridyl, pyrylium, thiopyrylium, pyrrolyl, furyl, thienyl, indolinyl, isoindolinyl, indolizinyl, imidazolyl, pyridonyl, pyronyl, pyrimidinyl, pyrazinyl, oxazolyl, thiazolyl, purinyl, quinolinyl, isoquinolinyl, quinoxalinyl, pyridazinyl, benzofuranyl, benzoxazolyl and acridinyl groups. Preferably, a heterocyclic group is a 3- to 18-membered, particularly a 3- to 14-membered and especially a 5- to 10-membered, ring system containing at least one heteroatom selected from oxygen, sulphur and nitrogen atoms. Preferred heterocyclic groups include the specific heteroaryl groups named above as well as pyranyl, piperidinyl, pyrrolidinyl, dioxanyl, piperazinyl, morpholinyl, tetrahydroisoquinolinyl and tetrahydrofuranyl groups.

When any of the foregoing substituents are designated as being optionally substituted, the substituent groups which are optionally present may be any one or more of those customarily employed in the development of pharmaceutical compounds and/or the modification of such compounds to influence their structure/activity, stability, bioavailability or other property. Specific examples of such substituents include, for example, halogen atoms, nitro, cyano, hydroxyl, cycloalkyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, arylsulphinyl, arylsulphonyl, arylsulphonato, carbamoyl and alkylamido groups. When any of the foregoing substituents represents or contains an alkyl substituent group, this may be linear or branched and may contain up to 12, preferably up to 6, and especially up to 4, carbon atoms. A cycloalkyl group may contain from 3 to 8, preferably from 3 to 6, carbon atoms. An aryl group or moiety may contain from 6 to 10 carbon atoms, phenyl groups being especially preferred. A halogen atom may be a fluorine, chlorine, bromine or iodine atom and any group which contains a halo moiety, such as a haloalkyl group, may thus contain any one or more of these halogen atoms.

It is preferred that R represents a hydrogen atom or an optionally substituted alkyl, aryl, aralkyl, heterocyclic or polycyclic group. Preferably, R represents a hydrogen atom or a C₁₋₆ alkyl, C₆₋₂₄ aryl, C₇₋₃₀ aralkyl, 5- to 18- membered heterocyclic or C₄₋₂₆ polycyclic group, each group being optionally substituted by one or more substituents selected form the group consisting of halogen atoms, hydroxyl, C₁₋₈ alkyl, C₁₋₄ haloalkyl, C₁₋₄alkoxy, C₁₋₄ haloalkoxy, carboxyl and C₁₋₄ alkoxycarbonyl groups. More preferably, R represents a hydrogen atom or a C₁₋₄ alkyl, C₆₋₁₄ aryl, C₇₋₁₈ aralkyl, 5- to 14- membered heterocyclic or C₆₋₁₈ polycyclic group, each group being optionally substituted by one or more substituents selected from the group consisting of halogen atoms, C₁₋₄ alkyl, C₁₋₄ haloalkyl, C₁₋₄ alkoxy and C₁₋₄ haloalkoxy groups.

In a particularly preferred sub-group of compounds, R represents a hydrogen atom or a methyl, trifluoroethyl, methoxyphenyl, naphthyl, benzyl, fluorobenzyl, naphthylmethyl, anthrylmethyl, phenanthrylmethyl, pyrylmethyl, quinolinyl, trifluoromethylquinolinyl or cholestenyl group. Compounds in which R represents a hydrogen atom or a methyl, fluorobenzyl, phenanthrylmethyl, pyrylmethyl or trifluoromethylquinolinyl group are particularly preferred.

Preferably, the parasite is an organism of the genus Neospora or the genus Eimeria.

The present invention also provides the use of a compound of the general formula I as defined above for the manufacture of a medicament for the treatment and/or prophylaxis of a disease caused by infection with a parasite other than an organism of a genus selected from the genera Toxoplasma, Entamoeba and Plasmodium. Preferably, the parasite is an organism of the genus Neospora or the genus Eimeria.

According to the present invention there is also provided a compound of the general formula I as defined above, with the proviso that R does not represent a hydrogen atom, methyl, ethyl, isopropyl, isobutyl or sec-butyl group, for use in the treatment and/or prophylaxis of a disease caused by infection with a parasite of the genus Toxoplasma. The present invention also provides the use of a compound of the general formula I as defined in the preceding paragraph for the manufacture of a medicament for the treatment and/or prophylaxis of a disease caused by infection with a parasite of the genus Toxoplasma.

According to another aspect of the present invention there is provided a compound of the general formula I as first defined above, with the proviso that R does not represent a 2-ethylbut-3-yn-2-yl or 3-(4-carboxylphenyl)isoxazol-5-ylmethyl group, for use in the treatment and/or prophylaxis of a disease caused by infection with a parasite of the genus Entamoeba. The use of such a compound of the general formula I for the manufacture of a medicament for the treatment and/or prophylaxis of a disease caused by infection with a parasite of the genus Entamoeba is also provided.

According to the present invention there is further provided a compound of the general formula I as defined in the ante-preceding paragraph, with the further proviso that R does not represent an n-propyl, n-butyl, iso-pentyl, tert-pentyl, n-octyl, 2-hydroxyethyl, 2-methoxyethyl, 1-ethanoylethyl, 1,3-bis(isopropoxy)prop-2-yl, prop-2-enyl, 3-phenylprop-2-enyl, but-3-yn-2-yl, 2-methylbut-3-yn-2-yl, 2-ethylbut-3-yn-2-yl, cyclopentyl, cyclohexyl, benzyl, 4-methoxybenzyl, 4-carboxylbenzyl, 4-(sodium-carboxylato)benzyl, 3-chloroisoxazol-5-ylmethyl, 3-bromoisoxazol-5-ylmethyl, 3- (4-carboxylphenyl)-isoxazol-5-ylmethyl, 2-(4-chlorophthalimido)ethyl or glucuronide group, for use in the treatment and/or prophylaxis of a disease caused by infection with a parasite of the genus Plasmodium.

In a particularly preferred sub-group of these compounds, R represents a trifluoroethyl, fluorobenzyl, naphthylmethyl, anthrylmethyl, phenanthrylmethyl, pyrylmethyl, quinolinyl or trifluoromethylquinolinyl group. 10β-(4'-Quinolinoxy)dihydroartemisinin and 10β-[4'-(7-'trifluoromethylquinolinoxy)]dihydroartemisinin have exhibited particularly interesting antimalarial activity.

The present invention also provides use of a compound of the general formula I as defined in the ante-preceding paragraph for the manufacture of a medicament for the treatment and/or prophylaxis of a disease caused by infection with a parasite of the genus Plasmodium.

Certain compounds of the general formula I are novel and the invention therefore further provides a compound of the general formula I as defined in the preceding paragraph, with the further proviso that R does not represent a 4-(methoxycarbonyl)benzyl, 4-(potassium carboxylato)benzyl or triethanoyloxyglucuronide group.

It should also be appreciated that the compounds of general formula I are capable of existing as different geometric and optical isomers. The present invention thus includes both the individual isomers and mixtures of such isomers.

The present invention also provides a process for the preparation of a novel compound of the general formula I as defined above which comprises reacting dihydroartemisinin with a compound of the general formula R-OH where R is as defined in the antepreceding paragraph.

The reaction of dihydroartemisinin with an alcohol of formula R-OH as defined above may be conveniently carried out in the presence of a solvent. Suitable solvents include ethers, especially aliphatic ethers, such as diethyl ether. The reaction may also be carried out in the presence of a Lewis acid, such as boron trifluoride dietherate, under an inert atmosphere, for instance, under nitrogen. Preferably, the reaction is carried out at room temperature, that is, 15 to 35°C, preferably 20 to 30°C.

The reaction of dihydroartemisinin with an alcohol of formula R-OH as defined above may also be conveniently carried out in the presence of a cyclic ether solvent such as tetrahydrofuran. In this case, the reaction may also be carried out in the presence of triphenylphosphine and diethyl azodicarboxylate under an inert atmosphere, for instance, under nitrogen. Preferably, the reaction is carried out at a temperature of -5°C to room temperature, preferably 0°C to room temperature. Ideally, the reagents are initially mixed together at 0°C and the reaction mixture is then allowed to warm to room temperature, that is, 15 to 35°C, preferably 20 to 35°C.

In some circumstances, it may be necessary to protect certain groups of the dihydroartemisinin and/or alcohol starting materials with a protecting group to reduce the likelihood of undesirable side reactions. Suitable protecting groups include trimethylsilyl groups. In such cases, the reaction is preferably carried out in a halogenated hydrocarbon solvent, particularly a chlorinated hydrocarbon, such as dichloromethane. Preferably, the reaction is carried out in the presence of a catalytic amount of trimethylsilyl trifluoromethanesulphonate (TMSOTf) solution under an inert atmosphere, such as a nitrogen atmosphere. Preferably, the reaction is carried out at a temperature of -70 to 80°C, especially about -78°C. Alternatively, this reaction may be carried out at a temperature of -5 to +5°C, especially about 0°C, if it is desired to increase the quantity of one of the isomers, usually the β-anomer, and to reduce the quantity of the side products obtained. The trimethylsilyl protected starting materials may be prepared by reacting the dihydroartemisinin and/or alcohol with a halotrimethylsilane, such as chlorotrimethylsilane, in the presence of a base, such as pyridine or triethylamine. Preferably, the reaction is carried out at room temperature, that is, 15 to 35°C, preferably 20 to 30°C.

Alternatively, if it is desired to increase the quantity of the other isomer, usually the α-anomer, the reaction of dihydroartemisinin with an alcohol of formula R-OH as defined above may be conveniently carried out in the presence of a halogenated hydrocarbon solvent, particularly a chlorinated hydrocarbon, such as dichloromethane, in the absence of any protecting groups. The reaction is preferably carried out under an inert atmosphere, such as a nitrogen atmosphere. Preferably, the reaction is carried out at a temperature of -30 to -10°C, particularly -25 to -15°C.

Dihydroartemisinin and the alcohols of formula R-OH, where R is defined above, are all known compounds or can be prepared by processes analogous to known processes.

The invention also provides a pharmaceutical composition which comprises a carrier and, as active ingredient, a novel compound of the general formula I as defined above.

A pharmaceutically acceptable carrier may be any material with which the active ingredient is formulated to facilitate administration. A carrier may be a solid or a liquid, including a material which is normally gaseous but which has been compressed to form a liquid, and any of the carriers normally used in formulating pharmaceutical compositions may be used. Preferably, compositions according to the invention contain 0.5 to 95% by weight of active ingredient.

The compounds of general formula I can be formulated as, for example, tablets, capsules, suppositories or solutions. These formulations can be produced by known methods using conventional solid carriers such as, for example, lactose, starch or talcum or liquid carriers such as, for example, water, fatty oils or liquid paraffins. Other carriers which may be used include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; polypeptide/protein or polysaccharide complexes such as gelatin-acacia complexes; sugars such as mannitol, dextrose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

Auxiliary components such as tablet disintegrants, solubilisers, preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include citric acid, tartaric acid, phosphoric acid, hydrochloric acid and maleic acid. Suitable sweeteners include aspartame, acesulfame K and thaumatin. Suitable taste-masking agents include sodium bicarbonate, ion-exchange resins, cyclodextrin inclusion compounds, adsorbates or microencapsulated actives.

For treatment of and prophylaxis against coccidiosis and related parasites, for instance, in poultry, especially in chickens, ducks, geese and turkeys, 0.1 to 100 ppm, preferably 0.5 to 100 ppm of the active compound may be mixed into an appropriate, edible material, such as nutritious food. If desired, the amounts applied can be increased, especially if the active compound is well tolerated by the recipient. Accordingly, the active compound can be applied with the drinking water.

For the treatment of a single animal, for instance, for the treatment of coccidiosis in mammals or toxoplasmosis, amounts of 0.5 to 100 mg/kg body weight active compound are preferably administered daily to obtain the desired results. Nevertheless, it may be necessary from time to time to depart from the amounts mentioned above, depending on the body weight of the experimental animal, the method of application, the animal species and its individual reaction to the drug or the kind of formulation or the time or interval in which the drug is applied. In special cases, it may be sufficient to use less than the minimum amount given above, whilst in other cases the maximum dose may have to be exceeded. For a larger dose, it may be advisable to divide the dose into several smaller single doses.

The invention also provides a method for treating a disease caused by infection with a parasite other than an organism of a genus selected from the genera Toxoplasma, Entamoeba and Plasmodium which comprises administering to a host in need of such treatment a therapeutically effective amount of a compound of the general formula I as first defined above. Preferably, the parasite is an organism of the genus Neospora or the genus Eimeria. A method for treating a disease caused by infection with a parasite of the genus Toxoplasma is also provided which comprises administering to a host in need of such treatment a therapeutically effective amount of a compound of the general formula I as defined above. The invention further provides a method for treating a disease caused by infection with a parasite of the genus Plasmodium which comprises administering to a host in need of such treatment a therapeutically effective amount of a compound as defined above. Preferably, the host is a mammalian host.

The invention is further illustrated by the following examples.

### Example 1

### Preparation of 10β-[(2',2',2'-trifluoroethyl)oxy]dihydroartemisinin (Formula I : R = -CH₂CF₃)

To a solution of dihydroartemisinin (426mg, 1.50 mmol) and 2,2,2-trifluoroethanol (220µl, 3.00 mmol) in diethyl ether (30 ml) at room temperature under nitrogen was added boron trifluoride dietherate (3 drops). The mixture was stirred at room temperature for 6 hours. The reaction was quenched with saturated NaHCO₃ solution, dried (MgSO₄) and concentrated in vacuo. Flash chromatography (SiO₂ : 8% ethyl acetate/hexanes) gave 10β-[(2',2',2'-trifluoroethyl)oxy]dihydroartemisinin (157mg, 29%) and 9,10-anhydrodehydroartemisinin (133mg, 46%) both as white solids.
Ether: M.p. 113.4 - 114.2°C; [αl_{D}²⁰ : +133° (c 0.30/CHCl₃); νₘₐₓ (film): 2984, 2960, 2928, 2870, 1454, 1414, 1378, 1308, 1278, 1174, 1150, 1110, 1052, 1036, 990, 972, 918, 874, 824; δ_{H} : 5.43 (1H, s, H-12), 4.90 (1H, d, J = 3.46 Hz, H-10), 4.15 (1H, dq, J = 12.2, 8.87 Hz, H-1'), 3.89 (1H, d, J = 12.2, 8.67 Hz, H-1'), 2.65-2.75 (1H, m, H-9), 2.40 (1H, ddd, J = 17.5, 13.4, 4.02 Hz), 2.07 (1H, ddd, J = 14.6, 4.83, 3.01 Hz), 1.64-1.96 (5H, m), 1.22-1.58 (4H, m), 1.46 (3H, s, H-14), 0.98 (3H, d, J = 5.94 Hz, H-16), 0.97 (3H, d, J = 7.69 Hz, H-15); δ_{C} : 104.13, 102.41, 87.93, 80.73, 64.91 (d, J = 34.3 Hz), 52.32, 43.99, 37.29, 36.17, 34.39, 30.44, 25.90, 24.47, 24.11, 20.13, 12.47; m/z (CI, NH₃): 384 (M+NH₄⁺, 16%), 367 (M⁺+1, 2), 284 (100), 267 (72).

### Example 2

### Preparation of 10β-[4'-(7'-trifluoromethylquinolinoxy)]dihydroartemisinin (Formula I : R = 7 - CF₃ quinolin-4-yl)

To a solution of dihydroartemisinin (286 mg, 1.00 mmol) and 7-trifluoromethyl-4-quinolinol (213mg, 1.00 mmol) in tetrahydrofuran (10 ml) was added triphenylphosphine (262 mg, 2.00 mmol) and diethyl azodicarboxylate (165 µl, 1.00 mmol) at 0°C under nitrogen. The mixture was stirred at room temperature overnight. The yellow solution was concentrated in vacuo. The residue was purified by flash chromatography (SiO₂: 40% ethyl acetate/hexanes) to give 10β-[4'-(7'-trifluoromethylquinolinoxy)]-dihydroartemisinin (315mg, 66%) as a white solid. The white solid was recrystallised from ethyl acetate-hexane mixture to give a white needle-shaped crystal. M.p. 270-272°C; [αl_{D}^{21.5} + 73.30° (c. 0.0179 in CHCl₃); νₘₐₓ (Nujol): 2922, 1596, 1570, 1510, 1456, 1378, 1332, 1302, 1194, 1162, 1132, 1098, 1064, 1040, 1014, 984, 950, 930, 898, 874, 828, 738, 684 cm⁻¹; δ_{H}: 7.39-8.91 (5H, m, Ar-H), 5.84 (1H, d, J = 3.35 Hz, H-10), 5.47 (1H, s, H-12), 2.99-3.07 (1H, m, H-9), 2.38-2.48 (1H, m), 1.69-2.23 (7H, m), 1.35-1.49 (3H, m), 1.49 (3H, s, H-14), 1.15 (3H, d, J = 7.35 Hz, H-15), 0.99 (3H, d, J = 5.67 Hz, H-16); δ_{F}: 61.09; m/z (CI, CH₄): 480 (M⁺+1, 12%), 460 (28), 267 (80), 221 (100), 214 (72), 163 (60).

### Example 3

### Preparation of 10α- and β-[(2'-naphthylmethyl)oxy]dihydroartemisinin (Formula I : R = -CH₂-(2-naphthyl)

### (a) Preparation of 10-(trimethylsiloxy)dihydroartemisinin

To a solution of dihydroartemisinin (1.51 g, 5.32 mmol) in pyridine (20 ml) at 0°C under nitrogen was added dropwise chlorotrimethylsilane (5.20 ml, mmol). The mixture was stirred at room temperature for a further 1 hour and poured into ice-water mixture. The solution was extracted with diethyl ether (3x15 ml), dried (MgSO₄) and concentrated in vacuo. The residue was purified by flash chromatography (SiO₂; 5% ethyl acetate/hexanes) to give 10-(trimethylsiloxy)dihydroartemisinin as a white solid (1.47 g, 78%). δ_{H} 5.49 (1H, s, H-12), 5.19 (1H, d, J = 3.05 Hz, H-10), 2.52-2.62 (1H, m, H-9), 2.39 (1H, ddd, J = 17.5, 13.4, 4.01 Hz), 2.04 (1H, ddd, J = 14.5, 4.84, 3.05 Hz), 1.20-1.97 (9H, m), 1.45 (3H, s, H-14), 0.97 (3H, d, J = 6.24 Hz, H-16), 0.87 (3H, d, J = 7.29 Hz, H-15), 0.17 (9H, s, (CH₃)₃Si).

### (b) Preparation of 10α- and β-[(2'-naphthylmethyl)oxy]dihydroartemisinin (Formula I : R = -CH₂-(2-naphthyl)

### Procedure 1 (at -78°C).

To a solution of 10-(trimethylsiloxy)dihydroartemisinin (238mg, 0.67 mmol) prepared as described in (a) above and 2-naphthylmethanol trimethylsilyl ether (170mg, 0.74 mmol) in distilled dichloromethane (8ml) was added a catalytic amount of trimethylsilyl trifluoromethanesulphonate (TMSOTf) solution (5µl TMSOTf in 1 ml dichloromethane) under a nitrogen atmosphere at -78°C. The reaction mixture was continuously stirred at -78°C for 18 hours, then it was diluted with dichloromethane (20ml), washed with sodium hydrogen carbonate solution (10%, 2 x 10 ml) and dried over anhydrous magnesium sulphate. After filtration of the magnesium sulphate, the filtrate was evaporated in vacuo. The two anomers were separated by repeated column chromatography (SiO₂, ether : hexane from 1:3 to 3:7). The β-anomer was obtained as a white foam (93 mg, 33%) and the α-anomer was obtained as a colourless viscous liquid (35 mg, 12%).

### Procedure 2 (at 0°C).

The same reaction with the same amount of reactants was performed at 0°C, the reaction mixture was stirred at 0°C for 2 hours, the α- and β- anomers were separated by column chromatography using the same solvent system. The α-anomer (30 mg, 11%) was the minor product and the β-anomer was the major product (237 mg, 84%). Raising the temperature of this reaction decreases the amount of the side products (dimers of the two reactants involved) or increases the yield of the anomers but changes the ratio of the 2 anomers.

### Procedure 3 (higher yield of the α-anomer).

To a solution of dihydroartemisinin (100 mg, 0.35 mmol) and 2-naphthylmethanol (61 mg, 0.39 mmol) in dichloromethane at -20°C under a nitrogen atmosphere was added a catalytic amount of chlorotrimethylsilane. The resulting mixture was stirred at -20°C until the reaction was complete. The reaction was then quenched with a saturated solution of NaHCO₃ the organic layer was isolated and dried. After evaporation of the solvent, the product was isolated by column chromatography (SiO₂, ether: hexane, from 2:8 to 3:7) to give a pair of anomeric ethers (28.8 mg, 78%) with α:β ratio of 1:3.
β-anomer: M.p. 51-52°C; [αl_{D}^{20.5} + 108.73° (c. 0.0102 in CHCl₃); νₘₐₓ (Neat): 3056, 2922, 2874, 1604, 1510, 1450, 1376, 1344, 1322, 1310, 1266, 1226, 1194, 1176, 1158, 1140, 1100, 1060, 1012, 956, 940, 876, 824, 738 cm⁻¹; ¹H nmr (300 MHz, CDCl₃, δ_{H} 7.38-7.84 (7H, m, Ar-H), 5.50 (1H, s, H-12), 5.04 (1H, d, J 12.36 Hz, Ha-1'), 4.95 (1H, d, J 3.45 Hz, H-10), 4.68 (1H, d, J 12.36 Hz, Hb-1'), 2.69 (1H, m, H-9), 2.38 (1H, dt, J 14.13, 4.17 Hz, H-8), 2.01 (1H, m, H-4), 1.18-1.91 (9H, m, H-2 x 2, H-3 x 2, H-5, H-6 x 2, H-7 x 2), 1.44 (3H, s, 1-CH₃), 0.95 (3H, d, J 7.35 Hz, 9-CH₃), 0.91 (3H, d, J 5.97 Hz, 5-CH₃) ppm; m/z (CI, NH₃): 441 ([M+NH₃]⁺,2), 378 ([M-3CH₃-1]⁺,27), 221 ([M-C₁₁H₉O-O₂-CH₃+1]⁺,100), 141 (71) ;
Anal.Calc. for C₂₆H₃₂O₅: 73.56; H, 7.60;
Found, C, 73.27; H, 7.64.
nOe-difference experiment: (300 MHz, CDCl₃) :
irradiation of the doublet signal of H-10 at δ 4.95 gave 9.4% enhancement in the multiplet signal of H-9 at δ 2.69, this showed that the stereochemistry of H-9 and H-10 are syn to each other.
α-anomer: [αl_{D}^{20.5} - 54.66° (c. 0.0118 in CHCl₃); νₘₐₓ (Neat): 3058, 2924, 1636, 1450, 1976, 1132, 1032, 878, 852, 824, 742 cm⁻¹; ¹H nmr (300 MHz, CDCl₃) δ_{H} 7.41-7.85 (7H, m, Ar-H), 5.34 (1H, s, H-12), 5.12 (1H, d, J 12.60 Hz, Ha-1'), 4.81 (1H, d, J 12.60 Hz, Hb-1'), 4.55 (1H, d, J 9.21 Hz, H-10), 2.55 (1H, m, H-9), 2.40 (1H, m, H-8), 2.05 (1H, m, H-4), 1.10-1.92 (9H, m, H-2 x 2, H-3 x 2, H-5, H-6 x 2, H-7 x 2), 1.48 (3H, s, 1-CH₃), 0.92 (6H, d x 2, J 7.35 Hz, 5-CH_{3'}, 9-CH₃) ppm; m/z (Cl, NH₃): 441 ([M+NH₃]⁺,1), 378 ([M-3CH₃-1]⁺,26), 267 ([M-C₁₁H₉O]⁺,18), 221 ([M-C₁₁H₉O-O₂-CH₃+1]⁺,100), 163 (13), 141 (88);
nOe difference experiment: (300 MHz, CDCl₃) :
irradiation of the doublet signal of H-10 at δ 4.55 gave 65% enhancement in the singlet signal of H-12 at δ 5.34, this showed that the stereochemistry of H-12 and H-10 are syn to each other.

### Examples 4 to 16

By processes similar to those described in Examples 1 to 3 above, further compounds according to the invention were prepared as detailed in Table I below. In this table, the compounds are identified by reference to formula I.

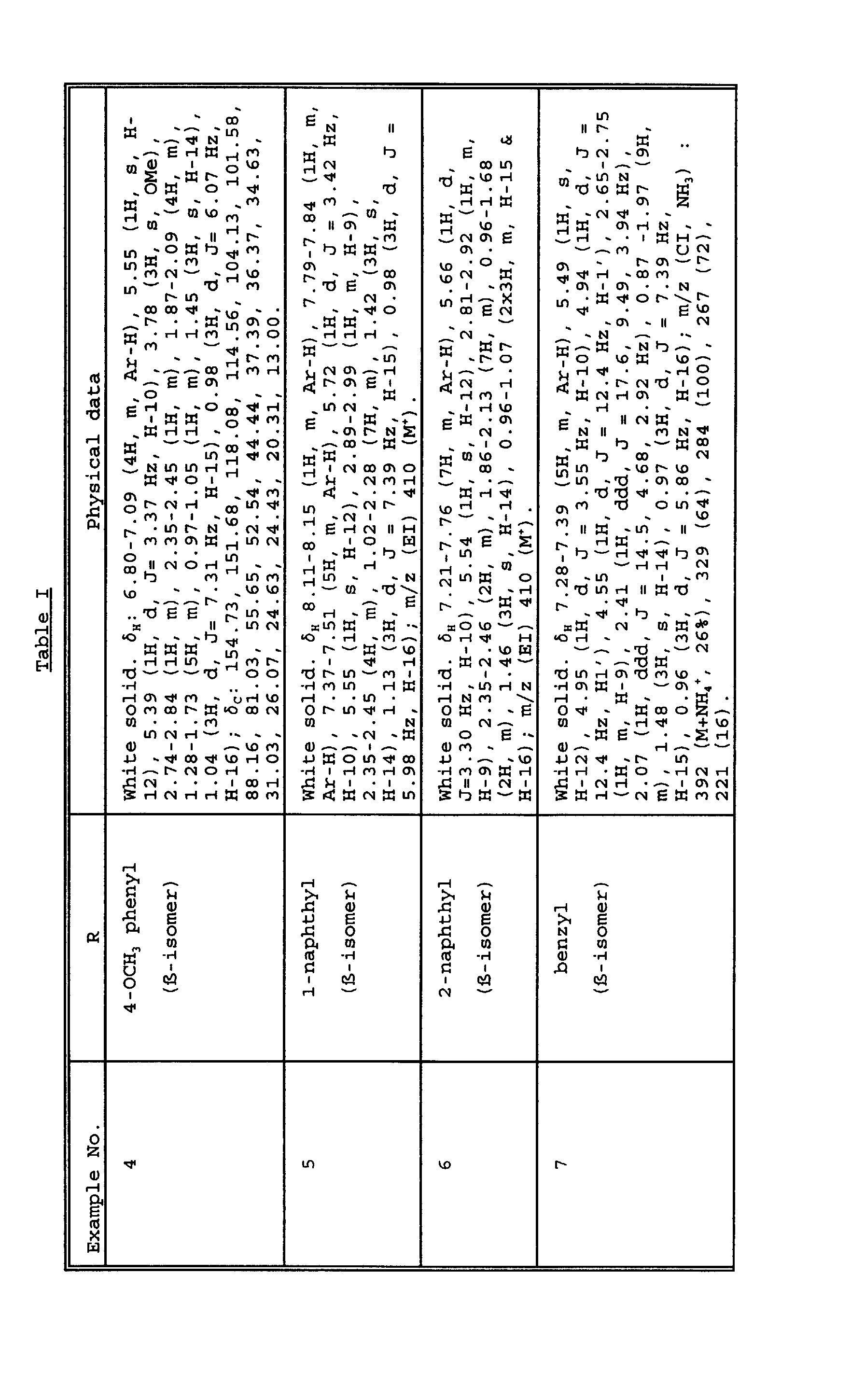

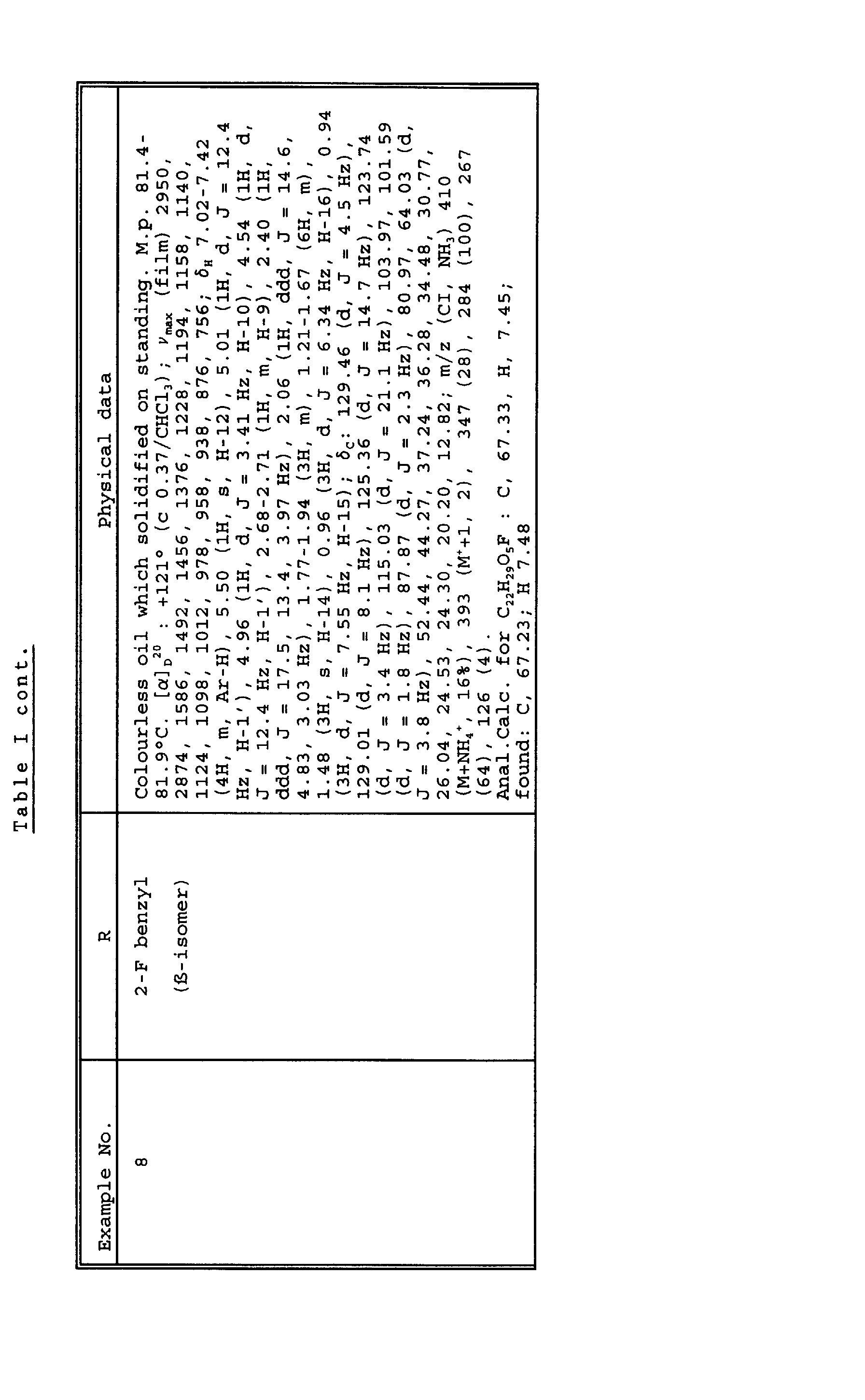

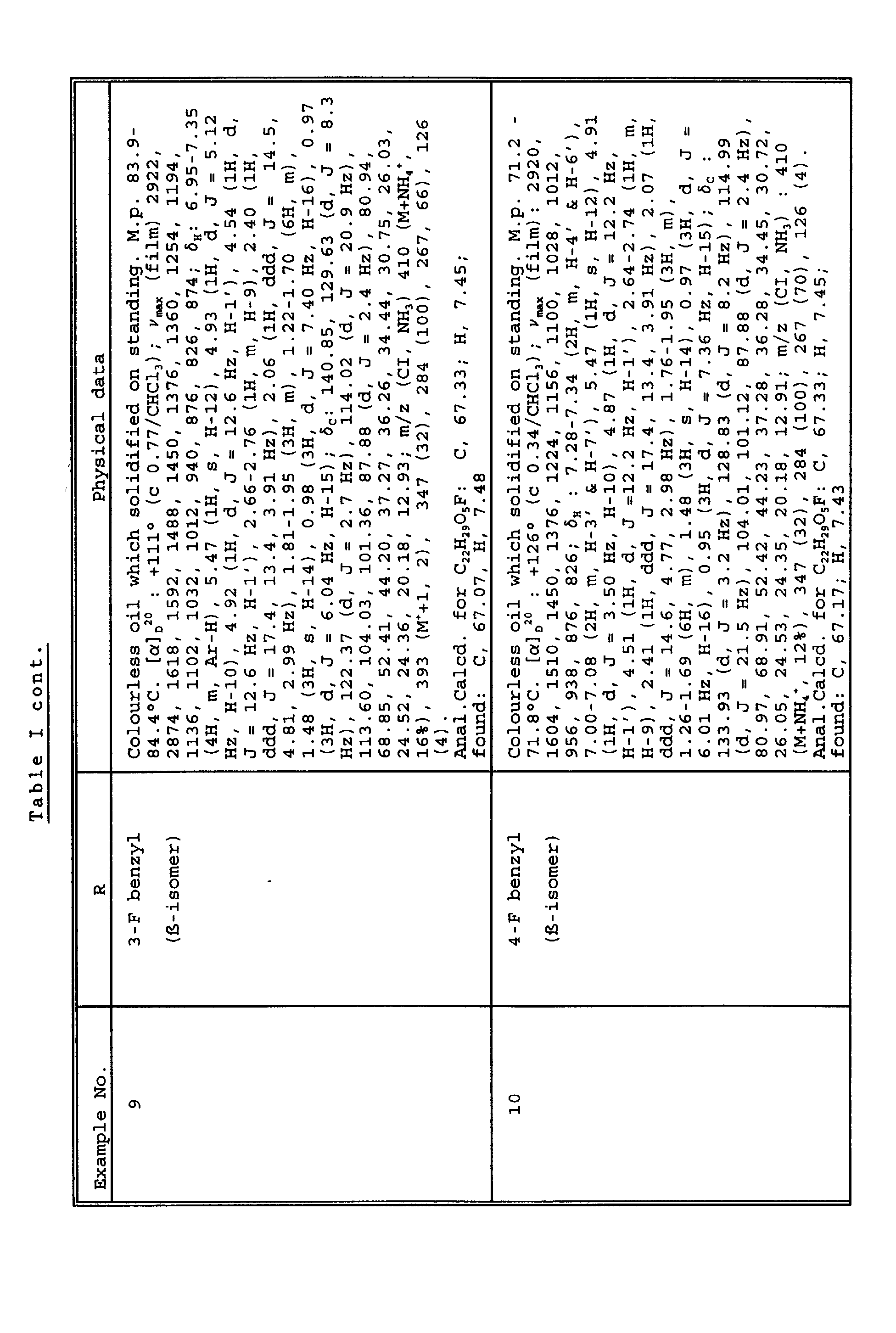

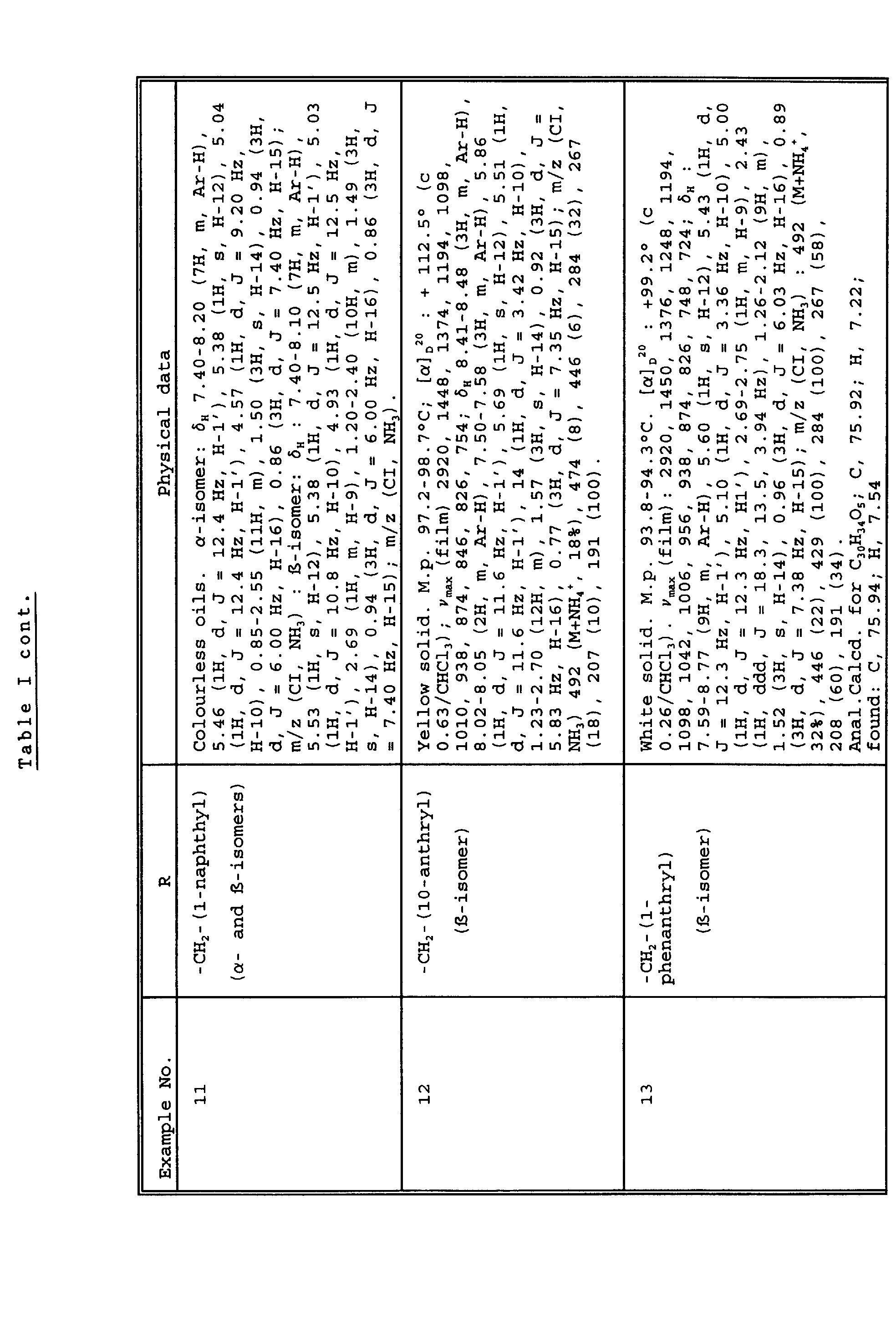

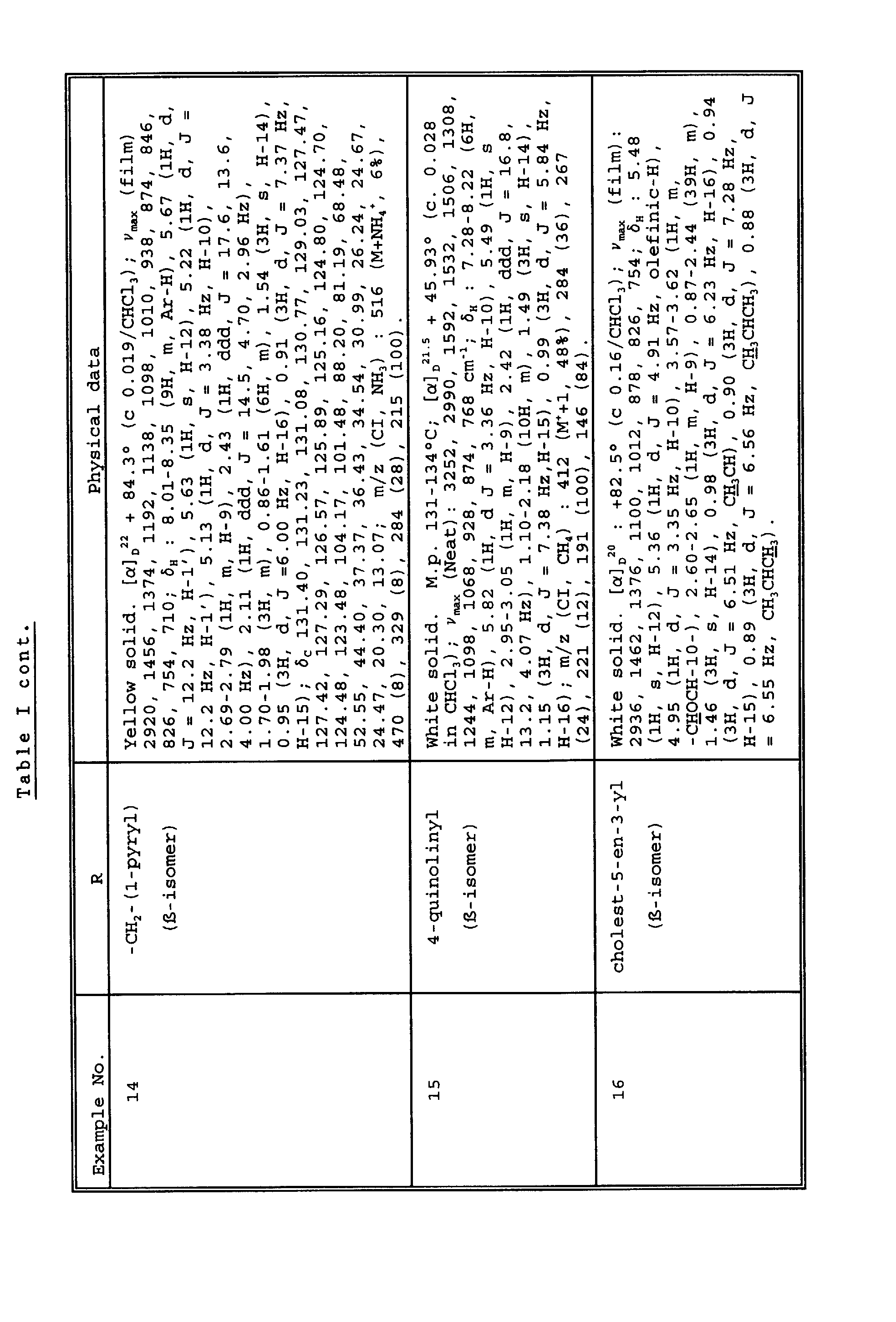

### Example 17

The parasiticidal activity of compounds of the invention was investigated by means of the following tests.
Abbreviations used in the examples:
- CO₂ =: carbon dioxide
- DMSO =: dimethylsulphoxide
- ED =: dermal cell line of a horse
- EDTA =: ethylenediaminetetraacetic acid
- FCS =: fetal calf serum
- RPMI =: growth medium for cell cultures
- rpm =: revolutions per minute
- VERO =: kidney cell line of the African green monkey

### (a) Screening of compounds against Neospora Caninum cell cultures in vitro.

Screening was conducted in 96-well plates (Falcon 3872). A monolayer of host cells (VERO or ED) were placed on a cell culture plate. Non-infected monolayers of cells were cultured in two 50 ml tissue culture bottles (50 cm³ cell culture area) . The cell layer was detached with trypsin-EDTA (5 ml. Gibco 45300-019) in a CO₂-culture cupboard at 37°C. After 10 minutes, most of the cells were detached. The cells were transferred with a 5 ml pipette into a 50 ml centrifuge tube (Greiner, B769331) containing about 1 ml warmed fetal calf serum. After centrifugation for 5 minutes at 1500 rpm (Varifuge 3.0, Heraeus), the liquid was removed and the cell pellet suspended in RPMI medium (100 ml, 95% RPMI 1640, 2% FCS, 1% L-glutamine, 1% sodium hydrogen carbonate, 1% penicillin/streptomycin). The cell suspension was pipetted into six 96-well plates at 150 µl per well. The coated cell culture plates were placed in an incubation cupboard at 37°C under 5% CO₂ for 24 hours. The cells were then infected with Neospora caninum tachyzoites at a concentration of 48,000 tachyzoites per well. This was followed by incubation at 37°C under 5% CO₂ for 24 hours.

The test compounds (0.5 - 1.5 mg) were weighed into 1.5 ml eppendorf vessels and dissolved in 1 ml dimethyl sulphoxide, corresponding to a dilution of about 1 x 10⁻³ g ml⁻¹. The medium used for further dilution consisted of 87% RPMI 1640, 10% FCS, 1% L-glutamine, 1% sodium hydrogen carbonate, 1% penicillin/streptomycin. In the first screening, concentrations of 10⁻⁵, 10⁻⁶ and 10⁻⁷ g ml⁻¹ were used. The diluted preparations were then transferred to the cell culture plates at a volume of 150 µl per well after 24 hour infection with Neospora caninum. For the first row, untreated medium was used; this row contained infected and uninfected cells as controls. The cell plate was incubated at 37°C under 5% CO₂ for 5 days. Microscopic evaluation was conducted 4 days after treatment and 5 days after infection at a magnification of 25 x 10 in an inverse microscope according to the following evaluation scheme.

| Evaluation | Observable effect |
|---|---|
| 0 = no effect | monolayer completely destroyed |
| 1 = weak effect | monolayer partly destroyed, parasite clumps can be seen |
| 2 = full effect | monolayer intact, no tachyzoites observable |
| T = cytotoxic | cells are dead, lysed |

The results are set out in Table II below:-

**Table II**

| Example No. | Dose (g/ml) | | | |
|---|---|---|---|---|
| | 10⁻⁵ | 10⁻⁶ | 10⁻⁷ | 10⁻⁸ |
| 2 | 2 | 0 | - | - |
| 8 | 1 | 1 | 0 | - |
| 9 | T/1 | 1 | 0 | - |
| 10 | T/2 | 1 | 0 | - |
| 13 | 1 | 1 | 0 | - |
| 14 | 1 | 0 | - | - |
| Dihydroartemisinin | 2 | 1 | 0 | - |
| Artemether | 1 | 0 | - | - |
| Artemisinin | 0 | - | - | - |

### (b) Screening of Compounds again Eimeria Tenella cell cultures in vitro

Cells from kidneys of 19 day old chicks are cultured as monolayers in 96-well plates (Falcon 3872) in a medium of Hanks lactalbumine hydrolysate, 5% fetal calf serum, 1% glutamine and 1% non-essential amino acids. After two days at 42°C under 5% CO₂, the culture was infected with excised sporozoites of Eimeria tenella at about 30.00 per well. Test compounds were dissolved in DMSO and diluted with culture medium to a maximum end concentration of 10µg ml⁻¹. The dilution steps were 1:10. On day 5 post infection, the cultures were evaluated under a microscope at 100-fold magnification and the condition of the host cells and the amount of intact schizonts and free merozoites was determined. Effectiveness was rated as follows:

| Evaluation | Observable effect |
|---|---|
| 3 = very active | no intact parasites/well |
| 2 = active | 1-6 parasites per well |
| 1 = weakly active | up to 1 intact schizont/optical field of vision |
| 0 = inactive | > 1 intact schizont/optical field of vision |
| T = cytotoxic | host cells are dead |

The results are set out in Table III below:-

**Table III**

| Example No. | Dose (g/ml) | | | |
|---|---|---|---|---|
| | 10⁻⁵ | 10⁻⁶ | 10⁻⁷ | 10⁻⁸ |
| 1 | 2 | 1 | 1 | 0 |
| 2 | 2 | 2 | 1 | 0 |
| 9 | T | T | 2 | 0 |
| 10 | T | 2 | 1 | 0 |
| 13 | T | T/2 | 2 | 0 |
| 14 | T | 2 | 1 | 0 |
| 16 | T/2 | 0 | - | - |
| Dihydroartemisinin | 2 | 2 | 0 | - |
| Artemether | 2 | 2 | 0 | - |
| Artemisinin | 2 | 1 | 0 | - |

### (c) In Vitro Screening against Plasmodium Falciparum

Two parasite strains - W2 resistant to chloroquine, and D6 sensitive to chloroquine but resistant to mefloquine were used. In Table IV below, the best compounds should show no cross resistance between the two strains.

The assay relies on incorporation of radiolabelled hypoxanthine by the parasite and inhibition of incorporation is attributed to activity of known or candidate antimalarial drugs. For each assay, proven antimalarials such as chloroquine, mefloquine, quinine, artemisinin and pyrimethamine were used as controls. The incubation period was 66 hours, and the starting parasitemia was 0.2% with 1% hematocrit. The medium was an RPMI-1640 culture with no folate or p-aminobenzoic acid. Albumax rather than 10% normal heat inactivated human plasma was used as, with Albumax, less protein binding is observed, and compounds elicit slightly higher activities in this model. If a compound was submitted with no prior knowledge of activity, it was dissolved directly in dimethyl sulphoxide (DMSO), and diluted 400 fold with complete culture medium. The unknown compound was started at a maximum concentration of 50,000 ng ml⁻¹ and sequentially diluted 2-fold for 11 times to give a concentration range of 1048 fold. These dilutions were performed automatically by a Biomek 1000 Liquid Handling System in 96-well microtiter plates. The diluted drugs were then transferred to test plates, 200 µl of parasitized erythrocytes were added, and incubated at 37°C in a controlled environment of 5% CO₂, 5% O₂ and 90% N₂. After 42 hours, 25 µl of ³H-hypoxanthine was added, and the plates incubated for an additional 24 hours. After 66 hours, the plates were frozen at -70°C to lyse the red cells, and then thawed and harvested onto glass fiber filter mats in a 96-well harvester. The filter mats were then counted in a scintillation counter. For each drug, the concentration response profile was determined and 50%, 90% and 10% inhibitory concentrations (IC₅₀, IC₉₀ and IC₁₀) were determined by a non-linear logistic dose response analysis program.

A prescreen format can be used wherein a 3-dilution assay may be used to determine activity at high medium or low concentrations. The concentrations were selected as 50,000, 500 and 50 ng ml⁻¹. These were performed in duplicate on a 96-well format plate with 14 test compounds and one known (standard) compound per plate. The system was automated with a Biomek diluter for mixing and diluting the drugs, and adding drugs and parasites to a test plate.

In the prescreen format, if the ANALYSIS FIELD (AF) has a "<", then the compound was "very active" and the IC values are most likely to be below the last dilution value (in nanograms/ml), which is listed next to AF. In most cases, these compounds were run again at lower starting concentration to determine the true IC value. If the AF has a ">", then the IC value is greater than the prescreen dilution value; thus "AF>250" means that the IC value is greater than 250 ng ml⁻¹ and no further screening is carried out. In such cases, values of 0.00 are entered for IC values.

The results are set out in Table IV below:-

**Table IV**

| Example No. | In vitro activity:IC₅₀;IC₉₀;(IC₁₀)ng/ml | |
|---|---|---|
| | W2 Strain (Chloroquine resistant) | D6 Strain (Chloroquine sensitive) |
| 1 | 0.26; 0.34; (0.19) | 0.77; 1.04; (0.57) |
| | 0.51; 1.28; (0.20) | |
| 2 | 0.67; 0.81; (0.56) | 0.71; 0.93; (0.55) |
| 3(α-isomer) | 1.54; 3.83; (0.62) | 1.24; 1.67; (0.93) |
| 3(β-isomer) | 0.64; 0.78; (0.53) | 0.40; 0.53; (0.31) |
| | 0.63; 1.25; (0.32) | 0.64; 0.80; (0.51) |
| 8 | 0.83; 1.08; (0.63) | 0.75; 1.22; (0.46) |
| 9 | 0.45; 0.59; (0.35) | 0.36; 1.70; (0.08) |
| 10 | 0.25; 0.86; (0.07) | 0.34; 0.58; (0.20) |
| 11(α-isomer) | 0.67; 1.01; (0.44) | 1.42; 1.97; (1.03) |
| | 0.70; 1.17; (0.42) | 1.47; 1.81; (1.20) |
| 12 | 131.64; 173.46; (99.90) | 135.83; 179.48; 102.79 |
| | 187.76; 301.34; (117.00) | |
| 13 | 0.62; 0.79; (0.50) | 0.58, 0.77; (0.44) |
| 14 | 17.21; 24.88; (11.90) | 37.94; 49.48; (29.09), |
| 15 | 1.24; 6.45; (0.24) | 0.43; 0.80; (0.23) |
| | 2.03; 9.11; (0.45) | 0.61; 0.91; (0.41) |
| 16 | 0.00; 0.00 5000 AF<5000 | 0.00;0.00 5000AF<5000 |

## Claims

1. A compound of the general formula I 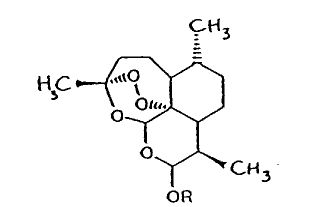 or a salt thereof
in which
R represents a hydrogen atom or an optionally substituted alkyl, alkenyl, alkynyl, cycloalkyl, aryl, aralkyl, heterocyclic or polycyclic group;
for use in the treatment and/or prophylaxis of a disease caused by infection with a parasite other than an organism of a genus selected from the genera Toxoplasma, Entamoeba and Plasmodium.

2. A compound according to claim 1 in which R represents a hydrogen atom or an optionally substituted alkyl, aryl, aralkyl, heterocyclic or polycyclic group.

3. A compound according to claim 1 or claim 2 in which R represents a hydrogen atom or a C₁₋₆ alkyl, C₆₋₂₄ aryl, C₇₋₃₀ aralkyl, 5- to 18- membered heterocyclic or C₄₋₂₆ polycyclic group, each group being optionally substituted by one or more substituents selected from the group consisting of halogen atoms, hydroxyl, C₁₋₈ alkyl, C₁₋₄ haloalkyl, C₁₋₄ alkoxy, C₁₋₄ haloalkoxy, carboxyl and C₁₋₄ alkoxycarbonyl groups.

4. A compound according to any one of the preceding claims in which R represents a hydrogen atom or a C₁₋₄ alkyl, C₆₋₁₄ aryl, C₇₋₁₈ aralkyl, 5- to 14- membered heterocyclic or C₆₋₁₈ polycyclic group, each group being optionally substituted by one or more substituents selected from the group consisting of halogen atoms, C₁₋₄ alkyl, C₁₋₄ haloalkyl, C₁₋₄ alkoxy and C₁₋₄ haloalkoxy groups.

5. A compound according to any one of the preceding claims in which R represents a hydrogen atom or a methyl, trifluoroethyl, methoxyphenyl, naphthyl, benzyl, fluorobenzyl, naphthylmethyl, anthrylmethyl, phenanthrylmethyl, pyrylmethyl, quinolinyl, trifluoromethylquinolinyl or cholestenyl group.

6. A compound according to any one of the preceding claims in which R represents a hydrogen atom or a methyl, trifluoroethyl, fluorobenzyl, phenanthrylmethyl, pyrylmethyl, trifluoromethylquinolinyl or cholestenyl group.

7. A compound according to any one of the preceding claims in which the parasite is an organism of the genus Neospora or the genus Eimeria.

8. Use of a compound of the general formula I as defined in any one of claims 1 to 6 for the manufacture of a medicament for the treatment and/or prophylaxis of a disease caused by infection with a parasite other than an organism of a genus selected from the genera Toxoplasma, Entamoeba and Plasmodium.

9. Use according to claim 8 in which the parasite is an organism of the genus Neospora or the genus Eimeria.

10. A compound of the general formula I as defined in any one of claims 1 to 6, with the proviso that R does not represent a hydrogen atom, methyl, ethyl, isopropyl, isobutyl or sec-butyl group, for use in the treatment and/or prophylaxis of a disease caused by infection with a parasite of the genus Toxoplasma.

11. Use of a compound of the general formula I as defined in claim 10 for the manufacture of a medicament for the treatment and/or prophylaxis of a disease caused by infection with a parasite of the genus Toxoplasma.

12. A compound of the general formula I as defined in claim 10, with the further proviso that R does not represent an n-propyl, n-butyl, iso-pentyl, tert-pentyl, n-octyl, 2-hydroxyethyl, 2-methoxyethyl, 1-ethanoylethyl, 1,3-bis(isopropoxy)prop-2-yl, prop-2-enyl, 3-phenylprop-2-enyl, but-3-yn-2-yl, 2-methylbut-3-yn-2-yl, 2-ethylbut-3-yn-2-yl, cyclopentyl, cyclohexyl, benzyl, 4-methoxybenzyl, 4-carboxylbenzyl, 4- (sodium carboxylato)benzyl, 3-chloroisoxazol-5-ylmethyl, 3-bromoisoxazol-5-ylmethyl, 3-(4-carboxylphenyl)isoxazol-5-ylmethyl, 2-(4-chlorophthalimido)ethyl or glucuronide group, for use in the treatment and/or prophylaxis of a disease caused by infection with a parasite of the genus Plasmodium.

13. A compound according to claim 12 in which R represents a trifluoroethyl, fluorobenzyl, naphthylmethyl, anthrylmethyl, phenanthrylmethyl, pyrylmethyl, quinolinyl or trifluoromethylquinolinyl group.

14. Use of a compound of the general formula I as defined in claim 12 or claim 13 for the manufacture of a medicament for the treatment and/or prophylaxis of a disease caused by infection with a parasite of the genus Plasmodium.

15. A compound of the general formula I as defined in claim 12, with the further proviso that R does not represent a 4-(methoxycarbonyl)benzyl, 4- (potassium carboxylato)benzyl or triethanoyloxyglucuronide group.

16. A process for the preparation of a compound of the general formula I according to claim 15 which comprises reacting dihydroartemisinin with a compound of the general formula R-OH where R is as defined in claim 15.

17. A pharmaceutical composition which comprises a carrier and, as active ingredient, a compound of the general formula I according to claim 15.

18. A method for treating a disease caused by infection with a parasite other than an organism of a genus selected from the genera Toxoplasma, Entamoeba and Plasmodium which comprises administering to a host in need of such treatment a therapeutically effective amount of a compound of the general formula I as defined in claim 1.

19. A method for treating a disease caused by infection with a parasite of the genus Toxoplasma which comprises administering to a host in need of such treatment a therapeutically effective amount of a compound of the general formula I as defined in claim 10.

20. A method for treating a disease caused by infection with a parasite of the genus Plasmodium which comprises administering to a host in need of such treatment a therapeutically effective amount of a compound of the general formula I as defined in claim 12.
